Europäisches Patentamt

European Patent Office

Office européen des brevets

(11) Publication number: 0 535 579 A1

# EUROPEAN PATENT APPLICATION

(21) Application number: 92116585.8

(22) Date of filing: 28.09.92

(51) Int. Cl.⁵: B26F 1/26

(30) Priority: 30.09.91 US 769047

(43) Date of publication of application:
07.04.93 Bulletin 93/14

(84) Designated Contracting States:
BE DE ES FR GB IT NL SE

(71) Applicant: KIMBERLY-CLARK CORPORATION
401 North Lake Street
Neenah Wisconsin 54957-0349(US)

(72) Inventor: Jameson, Lee Kirby
115 Olive Street
Roswell, Georgia 30075(US)
Inventor: Cohen, Bernard
381 Lakeshore Drive
Berkley Lake, Georgia 30136(US)

(74) Representative: Patentanwälte Grünecker,
Kinkeldey, Stockmair & Partner
Maximilianstrasse 58
W-8000 München 22 (DE)

(54) Hydrosonically microapertured thin thermoplastic sheet materials.

(57) A microapertured thin thermoplastic sheet material where the area of each of the microapertures is generally greater than about 10 square micrometers is disclosed.

FIGURE I

RELATED APPLICATIONS

This application is one of a group of applications which are being filed on the same date. It should be noted that this group of applications includes U.S. patent application serial number _____ entitled "Hydrosonically Microapertured Thin Thermoset Sheet Materials" in the names of Lee K. Jameson and Bernard Cohen; U.S patent application serial number _____ entitled "Hydrosonically Microapertured Thin Thermoplastic Sheet Materials" in the names of Bernard Cohen and Lee K. Jameson; U.S. patent application serial number _____ entitled "Pressure Sensitive Valve System and Process For Forming Said System" in the names of Lee K. Jameson and Bernard Cohen; U.S. patent application serial number _____ entitled "Hydrosonically Embedded Soft Thin Film Materials and Process For Forming Said Materials" in the names of Bernard Cohen and Lee K. Jameson; U.S. patent application number _____ entitled "Hydrosonically Microapertured Thin Naturally Occurring Polymeric Sheet Materials and Method of Making the Same" in the names of Lee K. Jameson and Bernard Cohen; U.S. patent application serial number _____ entitled "Hydrosonically Microapertured Thin Metallic Sheet Materials" in the names of Bernard Cohen and Lee K. Jameson; U.S. patent application serial number _____ entitled "Process For Hydrosonically Microaperturing Thin Sheet Materials" in the names of Lee K. Jameson and Bernard Cohen; and U.S. patent application serial number _____ entitled "Process For Hydrosonically Area Thinning Thin Sheet Materials" in the names of Bernard Cohen and Lee K. Jameson. All of these applications are hereby incorporated by reference.

FIELD OF THE INVENTION

The field of the present invention encompasses thin sheets formed from thermoplastic materials which have been microapertured in a generally uniform pattern.

BACKGROUND OF THE INVENTION

Ultrasonics is basically the science of the effects of sound vibrations beyond the limit of audible frequencies. Ultrasonics has been used in a wide variety of applications. For example, ultrasonics has been used for (1) dust, smoke and mist precipitation; (2) preparation of colloidal dispersions; (3) cleaning of metal parts and fabrics; (4) friction welding; (5) the formation of catalysts; (6) the degassing and solidification of molten metals; (7) the extraction of flavor oils in brewing; (8) electroplating; (9) drilling hard materials; (10) fluxless soldering and (10) nondestructive testing such as in diagnostic medicine.

The object of high power ultrasonic applications is to bring about some permanent physical change in the material treated. This process requires the flow of vibratory power per unit of area or volume. Depending on the application, the power density may range from less than a watt to thousands of watts per square centimeter. Although the original ultrasonic power devices operated at radio frequencies, today most operate at 20-69 kHz.

The piezoelectric sandwich-type transducer driven by an electronic power supply has emerged as the most common source of ultrasonic power; the overall efficiency of such equipment (net acoustic power per electric-line power) is typically greater than 70%. The maximum power from a conventional transducer is inversely proportional to the square of the frequency. Some applications, such as cleaning, may have many transducers working into a common load.

Other, more particular areas where ultrasonic vibratory force has been utilized are in the areas of thin nonwoven webs and thin films. For example, ultrasonic force has been use to bond or weld nonwoven webs. See, for example, U.S. patent numbers 3,575,752 to Carpenter, 3,660,186 to Sager et al., 3,966,519 to Mitchell et al. and 4,695,454 to Sayovitz et al. which disclose the use of ultrasonics to bond or weld nonwoven webs. U.S. patent numbers 3,488,240 to Roberts, describes the use of ultrasonics to bond or weld thin films such as oriented polyesters.

Ultrasonic force has also been utilized to aperture nonwoven webs. See, for example, U.S. patent numbers 3,949,127 to Ostermeier et al. and 3,966,519 to Mitchell et al..

Lastly, ultrasonic force has been used to aperture thin film material. See, for example, U. S. patent number 3,756,880 to Graczyk.

Other methods for the aperturing of thin film have been developed. For example, U.S. patent number 4,815,714 to Douglas discusses the aperturing of a thin film by first abrading the film, which is in filled and unoriented form, and then subjecting the film to corona discharge treatment.

One of the difficulties and obstacles in the use of ultrasonic force in the formation of apertures in materials is the fact that control of the amount of force which is applied was difficult. This lack of control resulted in the limitation of ultrasonic force to form large apertures as opposed to small microapertures. Such an application is discussed in U.K. patent application number 2,124,134 to Blair. One of the possible reasons that ultrasonics has not found satisfactory acceptance in the area of microaperture formation is that the amount of vibrational energy required to form a microaperture often resulted in a melt-through of the film.

As has previously been stated, those in the art had recognized that ultrasonics could be utilized to form apertures in nonwoven webs. See, U.S. patent to Mitchell. et al.. Additionally, the Mitchell et al. patent discloses that the amount of ultrasonic energy being subjected to a nonwoven web could be controlled by applying enough of a fluid to the area at which the ultrasonic energy was being applied to the nonwoven web so that the fluid was present in uncombined form. Importantly, the Mitchell, et al. patent states that the fluid is moved by the action of the ultrasonic force within the nonwoven web to cause aperture formation in the web by fiber rearrangement and entanglement. The Mitchell et al. patent also states that, in its broadest aspects, since these effects are obtained primarily through physical movement of fibers, the method of their invention may be utilized to bond or increase the strength of a wide variety of fibrous webs.

While the discovery disclosed in the Mitchell et al. patent, no doubt, was an important contribution to the art, it clearly did not address the possibility of aperturing nonfibrous sheets or sheets having fixed fibers formed from thermoplastic materials. This fact is clear because the Mitchell et al. patent clearly states the belief that the mechanism of aperture formation depended upon fiber rearrangement. Of course, such sheet materials either do not have fibers or have fibers which are in such a condition that they cannot be rearranged. Accordingly, it can be stated with conviction that the applicability of a method for aperturing such thermoplastic sheet materials by the application of ultrasonic energy in conjunction with a fluid at the point of application of the ultrasonic energy to the thermoplastic sheet material was not contemplated by the Mitchell et al. patent. Moreover, the Mitchell et al. patent teaches away from such an application because the patent states the belief that aperture formation requires the presence of movable fibers to be rearranged.

DEFINITIONS

As used herein the term "thermoplastic material" refers to a high polymer that softens when exposed to heat and returns to its original condition when cooled to room temperature. Natural substances which exhibit this behavior are crude rubber and a number of waxes. Other exemplary thermoplastic materials include, without limitation, polyvinyl chloride, polyesters, nylons, fluorocarbons, linear polyethylene such as linear low density polyethylene, polyurethaneprepolymer, polystyrene, polypropylene, polyvinyl alcohol, caprolactams, and cellulosic and acrylic resins.

As used herein the term "thermoplastic sheet material" refers to a generally nonporous item formed from a thermoplastic material that can be arranged in generally planar configuration. If the material is not a water soluble material, the material, in an unapertured state prior to being modified in accordance with the present invention, has a hydrostatic pressure (hydrohead) of at least about 100 centimeters of water when measured in accordance with Federal Test Method NO. 5514, standard no. 191A. Unless otherwise stated herein all hydrohead values are obtained in accordance with Federal Test Method NO. 5514, standard no 191A. This term is also intended to include multilayer materials which include at least one such sheet of a thermoplastic material as a layer thereof. Of course, if the thermoplastic material is water soluble, hydrohead measurements, if obtainable, have little, if any meaning.

As used herein tee term "thin thermoplastic sheet material" refers to a thermoplastic sheet material having an average thickness generally of less than about ten (10) mils. Average thickness is determined by randomly selecting five (5) locations on a given sheet material, measuring the thickness of the sheet material at each location to the nearest 0.1 mil, and averaging the five values (sum of the five values divided by five).

As used herein the term water vapor transmission rate (wvtr) refers to the rate water vapor will pass through a water insoluble sheet material under a given set of conditions in a particular time period. Unless otherwise specified, water vapor transmission rate is measured in accordance with ASTM E 96-80 using the water method referenced at paragraph 3.2 thereof. The test is run at 90 degrees fahrenheit and 50 percent relative humidity for twenty-four (24) hours.

As used herein the term "mesh count" refers to the number which is the product of the number of wires in a wire mesh screen in both the machine (MD) and cross-machine (CD) directions in a given unit area. For example, a wire mesh screen having 100 wires per inch in the machine direction and 100 wires per inch in the cross machine direction would have a mesh count of 10,000 per square inch. As a result of the interweaving of these wires, raised areas are present on both sides of the mesh screen. The number of raised areas on one side of such a wire mesh screen is generally one-half of the mesh count.

As used herein the term "aperture" refers to a generally linear hole or passageway. Aperture is to be distinguished from and does not include holes or passageways having the greatly tortuous path or passageways found in membranes.

As used herein the term "microaperture" refers to an aperture which has an area of less than about 100,000 square micrometers. The area of the microaperture is to be measured at the narrowest point in the linear passageway or hole.

As used herein the term "ultrasonic vibrations" refers to vibrations having a frequency of at least about 20,000 cycles per second. The frequency of the ultrasonic vibrations may range from about 20,000 to about 400,000 cycles per second or more.

As used herein the term "hydrosonics" refers to the application of ultrasonic vibrations to a material where the area of such application is has had a liquid applied thereto to the extent that the liquid is present in sufficient quantity to generally fill the gap between the tip of the ultrasonic horn and the surface of the material.

The approximate edge length of the thin microapertured thermoplastic sheet material of the present invention is calculated from the size of the microaperture using the appropriate geometrical formula, depending upon the microaperture's general shape.

OBJECTS OF THE INVENTION

Accordingly, it is a general object of the present invention to provide thin thermoplastic sheet materials which have been microapertured in a generally uniform pattern.

Still further objects and the broad scope of applicability of the present invention will become apparent to those of skill in the art from the details given hereinafter. However, it should be understood that the detailed description of the presently preferred embodiments of the present invention is given only by way of illustration because various changes and modifications well within the spirit and scope of the invention will become apparent to those of skill in the art in view of this detailed description.

SUMMARY OF THE INVENTION

In response to the foregoing problems and difficulties encountered by those in the art, we have developed a method for forming microapertures in a thin thermoplastic sheet material having a thickness of about 10 mils or less where the area of each of the formed microapertures is generally greater than about 10 square micrometers. The method includes the steps of: (1) placing the thin thermoplastic sheet material on a pattern anvil having a pattern of raised areas where the height of the raised areas is greater than the thickness of the thin thermoplastic sheet material; (2) conveying the thin thermoplastic sheet material, while placed on the pattern anvil, through an area where a fluid is applied to the thin thermoplastic sheet material; and (3) subjecting the thin thermoplastic sheet material to ultrasonic vibrations in the area where the fluid is applied to the thin thermoplastic sheet material. As a result of this method, the thin thermoplastic sheet material is microapertured in a pattern generally the same as the pattern of raised areas on the pattern anvil.

The thin thermoplastic sheet material may be formed from, for example, a material selected from the group including of one or more of polyolefins; such as, for example, linear low density polyethylene; polyesters; nylons; polyvinyl alcohol; caprolactams; or thermoplastic elastomers such as, for example, polyurethanes.

The fluid may be selected from the group including one or more of water; mineral oil; a chlorinated hydrocarbon; ethylene glycol; or a solution of 50 volume percent water and 50 volume percent 2 propanol. The chlorinated hydrocarbon may be 1,1,1 trichloroethane or carbon tetrachloride.

In some embodiments, the area of each of the formed microapertures may generally range from at least about 10 square micrometers to about 100,000 square micrometers. For example, the area of each of the formed microapertures may generally range from at least about 10 square micrometers to about 5,000 square micrometers. More particularly, the area of each of the formed microapertures may generally range from at least about 10 square micrometers to about 1,000 square micrometers. Even more particularly, the area of each of the formed microapertures may generally range from about at least 10 square micrometers to about 100 square micrometers.

The thin thermoplascic sheet material may be microapertured with a microaperture density of at least about 1,000 microapertures per square inch. For example, the thin thermoplastic sheet material may be microapertured with a microaperture density of at least about 5,000 microapertures per square inch. More particularly, the thin thermoplastic sheet material may be microapertured with a microaperture density of at least about 20,000 microapertures per square inch. Even more particularly, the thin thermoplastic sheet material may be microapertured with a microaperture density of at least about 90,000 microapertures per square inch. Yet even more particularly, the thin thermoplastic sheet material may be microapertured with a microaperture density of at least about 160,000 microapertures per square inch.

In some embodiments it may be desirable for the microaperturing of the thin thermoplastic sheet material to be confined to a predesignated area or areas of the thin thermoplastic sheet material. This result may be obtained where only a portion of the thin thermoplastic sheet is subjected to ultrasonic vibrations. Alternatively, this result may be obtained where only a portion of the pattern anvil is provided with raised areas.

The thickness of the thin thermoplastic sheet material is at least about 0.25 mil. For example, the thickness of the thin thermoplastic sheet material may range from about 0.25 mil to about 5 mils. More particularly, the thickness of the thin thermoplastic sheet material may range from about 0.25 mil to about 2 mils. Even more particularly, the thickness of the thin thermoplastic sheet material may range from about 0.5 mil to about 1 mil.

If it is not a water soluble material, the hydrohead of the thin microapertured thermoplastic sheet material may range from at least about 15 centimeters of water. For example, the hydrohead of the thin microapertured thermoplastic sheet material may range from at least about 35 centimeters of water. More particularly, the hydrohead of the thin microapertured thermoplastic sheet material may range from at least about 45 centimeters of water. Even more particularly, the hydrohead of the thin microapertured thermoplastic sheet material may range from at least about 55 centimeters of water. For example, the hydrohead of the thin microapertured thermoplastic sheet material may range from at least about 75 centimeters of water.

If it is not a water soluble material, the water vapor transmission rate of the thin thermoplastic sheet material may range from at least about 200 grams per square meter per day. For example, the water vapor transmission rate of the thin thermoplastic sheet material may range from at least about 500 grams per square meter per day. Even more particularly, the water vapor transmission rate of the thin thermoplastic sheet material may range from at least about 1,000 grams per square meter per day.

As a result of the microaperturing process the edge length of the thin thermoplastic sheet material may be increased by at least about 100 percent as compared to the sheet's edge length prior to microaperturing. For example, the edge length of the thin thermoplastic sheet material may be increased by at least about 500 percent as compared to the sheet's edge length prior to microaperturing. More particularly, the edge length of the thin thermoplastic sheet material may be increased by at least about 1,500 percent as compared to the sheet's edge length prior to microaperturing. Even more particularly, the edge length of the thin thermoplastic sheet material may be increased by at least about 3,000 percent as compared to the sheet's edge length prior to microaperturing.

## THE FIGURES

Figure I is a schematic representation of apparatus which utilizes ultrasonic vibrations to microaperture thin thermoplastic sheet materials.

Figure II is a cross sectional view of the transport mechanism for transporting the thin thermoplastic sheet material to the area where it is subjected to ultrasonic vibrations.

Figure III is a detailed view of the area where the thin thermoplastic sheet material is subjected to ultrasonic vibrations. The area is designated by the dotted circle in Figure I.

Figure IV is a photomicrograph of a 0.5 mil thick sheet of polyethylene film obtained from the Edison Company of Plainfield, New Jersey under the trade name "S/E 702", which has been microapertured in accordance with the present invention. The photomicrograph is accompanied by a scale where each unit on the scale represents ten microns.

## DETAILED DESCRIPTION OF THE INVENTION

Turning now to the figures where like reference numerals represent like structure and, in particular to Figure I which is a schematic representation of an apparatus which can carry out the method of the present invention, it can be seen that the apparatus is generally represented by the reference numeral 10. In operation, a supply roll 12 of a thin thermoplastic sheet material 14 to be microapertured is provided. As has been previously stated, the term thin thermoplastic sheet material refers to sheet materials which have an average thickness of about ten (10) mils or less. Additionally, generally speaking, the average thickness of the thin thermoplastic sheet material 14 will be at least about 0.25 mil. For example, the average thickness of the thin thermoplastic sheet 14 material may range from about 0.25 mil to about 5 mils. More particularly, the average thickness of the thin thermoplastic sheet material 14 may range from about 0.25 mil to about 2 mils. Even more specifically, the average thickness of the thin thermoplastic sheet material 14 may range from about 0.5 mil to about 1 mil.

The thin thermoplastic sheet material 14 may be formed from a material selected from one or more of, for example, a material selected from the group including of one or more of polyolefins; such as, for example, linear low density polyethylene; polyesters; nylons; or thermoplastic elastomers such as, for example, polyurethanes. The thin thermoplastic sheet material 14 may be formed from a blend of one or more thermoplastic materials which may be combined to form the sheet material 14.

The thin thermoplastic sheet material 14 is transported to a first nip 16 formed by a first transport roll 18 and a first nip roller 20 by the action of an endless transport mechanism 22 which moves in the direction indicated by the arrow 24. The transport mechanism 22 is driven by the rotation of the first transport roller 18 in conjunction with a second transport roller 26 which, in turn are driven by a conventional power source, not shown.

Figure II is a cross sectional view of the transport mechanism 22 taken along lines A-A in Figure I. Figure II discloses that the transport mechanism 22 includes a heavy duty transport wire mesh screen 28 usually having a mesh count of less than about 400 (i.e. less than a 20 wires per inch MD by 20 wires per inch CD mesh screen if machine direction (MD) and cross machine direction (CD) wire count is the same). Heavy duty mesh wire screens of this type may be made from a variety of materials such as, for example, plastics, nylons or polyesters, and are readily available to those in the art. Located above and attached to the transport screen 28 is an endless flat shim plate 30. The shim plate 30 desirably is formed from stainless steel. However, those of skill in the art will readily recognize that other materials may be utilized. Located above and attached to the shim plate 30 is a fine mesh wire pattern screen 32 usually having a mesh count of at least about 2,000 (i.e. at least about 45 wires per inch MD by 45 wires per inch CD mesh screen if MD and CD wire count is the same). Fine mesh wire screens of this type are readily available to those in the art. The fine mesh wire screen 32 has raised areas or knuckles 34 which perform the function of a pattern anvil as will be discussed later.

From the first nip 16 the thin thermoplastic sheet material 14 is transported by the transport mechanism 22 over a tension roll 36 to an area 38 (defined in Figure I by the dotted lined circle) where the thin thermoplastic sheet material 14 is subjected to ultrasonic vibrations.

The assembly for subjecting the thin thermoplastic sheet material 14 to the ultrasonic vibrations is conventional and is generally designated at 40. The assembly 40 includes a power supply 42 which, through a power control 44, supplies power to a piezoelectric transducer 46. As is well known in the art, the piezoelectric transducer 46 transforms electrical energy into mechanical movement as a result of the transducer's vibrating in response to an input of electrical energy. The vibrations created by the piezoelectric transducer 46 are transferred, in conventional manner, to a mechanical movement booster or amplifier 48. As is well known in the art, the mechanical movement booster 48 may be designed to increase the amplitude of the vibrations (mechanical movement) by a known factor depending upon the configuration of the booster 48. In further conventional manner, the mechanical movement (vibrational energy) is transferred from the mechanical movement booster 48 to a conventional knife edge ultrasonic horn 50. It should be realized that other types of ultrasonic horns 50 could be utilized. For example, a rotary type ultrasonic horn could be used. The ultrasonic horn 50 may be designed to effect yet another boost or increase in the amplitude of the mechanical movement (vibrations) which is to be applied to the thin thermoplastic sheet material 14. Lastly, the assembly includes an actuator 52 which includes a pneumatic cylinder, not shown. The actuator 52 provides a mechanism for raising and lowering the assembly 40 so that the tip 54 of the ultrasonic horn 50 can apply tension to the transport mechanism 22 upon the assembly 40 being lowered. It has been found that it is necessary to have some degree of tension applied to the transport mechanism 22 upon the lowering of the assembly for proper application of vibrational energy to the thin sheet material 14 to form microapertures in the thin thermoplastic sheet material 14. One desirable aspect of this tensioned arrangement is that the need to design a finely toleranced gap between the tip 54 of the horn 50 and the raised areas or knuckles 34 of the fine mesh wire screen 32 is not necessary.

Figure III is a schematic representation of the area 38 where the ultrasonic vibrations are applied to the thin thermoplastic sheet material 14. As can be seen in Figure III, the transport mechanism 22 forms an angle 56 with the tip 54 of the ultrasonic horn 50. While some microaperturing will occur if the angle 56 is as great as 45 degrees, it has been found that it is desirable for the angle 56 to range from about 5 degrees to about 15 degrees. For example, the angle 56 may range from about 7 to about 13 degrees. More particularly, the angle 56 may range from about 9 to about 11 degrees.

Figure III also illustrates that the transport mechanism 22 is supported from below by the first tension roll 36 and a second tension roll 58. Positioned somewhat prior to the tip 54 of the ultrasonic horn 50 is a spray nozzle 60 which is configured to apply a fluid 62 to the surface of the thin thermoplastic sheet material 14 just prior to the sheet material's 14 being subjected to ultrasonic vibrations by the tip 54 of the ultrasonic horn 50. The fluid 62 desirably may be selected from the group including one or more of water; mineral oil; a chlorinated hydrocarbon; ethylene glycol; or a solution of 50 volume percent water and 50 volume percent 2 propanol. For example, in some embodiments the chlorinated hydrocarbon may be selected from the group including 1,1,1 trichloroethane or carbon tetrachloride. It should be noted that the wedge-shaped area 64 formed by the tip 54 of the ultrasonic horn 50 and the transport mechanism 22 should be subjected to a sufficient amount of the fluid 62 for the fluid 62 to act as both a heat sink and a coupling agent for the most desirable results. Positioned below the transport mechanism 22 in the area where the tip 54 of the ultrasonic horn 50 is located is a fluid collection tank 66. (See Figure I.) The fluid collection tank 66 serves to collect fluid 62 which has been applied to the surface of the thin thermoplastic sheet material 14 and which has either been driven through the sheet material 14 and/or the transport mechanism 22 or over the edges of the transport mechanism 22 by the action of the vibrations of the tip 54 of the ultrasonic horn 50. Fluid 62 which is collected in the collection tank 66 is transported by tubing 68 to a fluid holding tank 70.

Figure I illustrates that the fluid holding tank 70 contains a pump 72 which, by way of additional tubing 74, supplies the fluid 62 to the fluid spray nozzle 60. Accordingly, the fluid 62 may be re-cycled for a considerable period of time.

While the mechanism of action may not be fully understood and the present application should not be bound to any particular theory or mechanism of action, it is believed that the presence of the fluid 62 in the wedge-shaped area 64 during operation of the ultrasonic horn 50 accomplishes two separate and distinct functions. First, the presence of the fluid 62 allows the fluid 62 to act as a heat sink which allows the ultrasonic vibrations to be applied to the thin thermoplastic sheet material 14 without the thin thermoplastic sheet material 14 being altered or destroyed as by melting. Secondly, the presence of the fluid 62 in the wedge-shaped area 64 allows the fluid 62 to act as a coupling agent in the application of the vibrations from the ultrasonic horn 50 to the thin thermoplastic sheet material 14.

It has been discovered that the action of the ultrasonic horn 50 on the thin thermoplastic sheet material 14 microapertures the thin thermoplastic sheet material 14 in spite of the fact that there are no fibers to re-arrange to form microapertures as was the case in Mitchell et al.. The microapertures are punched through the thin thermoplastic sheet material 14 in the pattern of the raised areas or knuckles 34 of the fine mesh wire pattern screen 32. Generally, the number of microapertures produced will be equal to the number of raised areas or knuckles 34 on the upper surface of the fine mesh wire screen 32. That is, the number of microapertures will generally be one-half the mesh count of a given area of pattern screen 32. For example, if the pattern screen 32 is 100 wires per inch MD by 100 wires per inch CD, the total number of knuckles or raised areas 34 on one side of the pattern wire 32, per square inch, will be 100 times 100 divided by 2. This equals 5,000 microapertures per square inch. For a 200 wires per inch MD by 200 wires per inch CD pattern screen 32 the calculation yields 20,000 microapertures per square inch. Depending somewhat on the thickness of the thin thermoplastic sheet material 14, at a mesh count of about 90,000 (300 wires per inch MD by 300 wires per inch CD) the wires are so thin as to allow the knuckles 34 on both sides to microaperture the thin thermoplastic sheet material 14 if sufficient force is applied. Thus, a 300 wires per inch MD by 300 wires per inch CD mesh screen yields 90,000 microapertures per square inch; for a 400 wires per inch MD by 400 wires per inch CD mesh--160,000 microapertures per square inch. Of course the MD and CD wire count of the wire mesh screen does not have to be the same.

It should also be noted that the number of microapertures formed may also vary with the number of ultrasonic vibrations to which the thin thermoplastic sheet material 14 is subjected per unit area for a given period of time. This factor may be varied in a number of ways. For example, the number and size of the microapertures will vary somewhat with the line speed of the thin thermoplastic sheet material 14 as it passes underneath the tip 54 of the ultrasonic horn 50. Generally speaking, as line speed increases, first the size of the microapertures decreases and then the number of microapertures decreases. As the number of microapertures decreases the less the pattern of microapertures resembles the pattern of raised areas 34 on the pattern screen 32. The range of line speeds that usually yields microapertures varies with the thermoplastic material utilized to form the thin thermoplastic sheet material 14 and the material used as the fluid 62. For polyethylene having a thickness of about 0.5 mil, typical line speeds which usually yield microapertures for a wide variety of fluids range from about 5 to about 25 feet per minute. For example, if water is used as the fluid with polyethylene typical line speeds which usually yield microapertures range from about 5 to about 23 feet per minute. It is believed that, to some extent, the variations in the number of microapertures formed and the size of the microapertures occurs due to the minute variations in the height of the raised areas or knuckles 34 of the fine mesh pattern screen 32. It should be noted that the fine mesh pattern screens used to date have been obtained from conventional everyday sources such as a hardware store. It is also believed that if a pattern screen 32 could be created where all of the raised areas 34 of the screen 32 were of exactly the same height these variations would only occur in uniform fashion with variations of line speed.

As was stated above, the area or size of each of the microapertures formed will also vary with the parameters discussed above. The area of the microapertures will also vary with the area of the raised areas of the pattern anvil such as the knuckles 34 on the fine mesh wire screen 32. It is believed that the type of thermoplastic material used in forming the thin thermoplastic sheet material 14 will also vary the area of the microapertures formed if all other parameters are maintained the same. For example, the softer the thin thermoplastic sheet material 14, the easier it is to push the thin thermoplastic sheet material 14 through the raised areas of the fine mesh pattern screen 32. Because the raised areas (knuckles) on the fine mesh screen are generally pyramidal in shape, the deeper the raised area penetrates the thin thermoplastic sheet material 14, the larger the microaperture. In such situations the shape of the microaperture will conform generally to the pyramidal shape of the raised area of the fine mesh screen and the microaperture will be generally pyramidally shaped, in the z direction, and will have an area which is greater at one end than at the other. As has been previously stated, the area of the microaperture should be measured at the narrowest point of the aperture. Of course, the height of the raised areas must be greater than the thickness of the thin sheet material 14 for microapertures to be formed and the degree of excess, if any, necessary may vary with the type of thermoplastic sheet to be microapertured. In any event, the height of the raised areas must be sufficient to punch through the thermoplastic material including any elasticity which might be encountered in the punching operation. That is, the more elastic the thermoplastic material, the greater the height of the raised areas has to exceed the thickness of the thin thermoplastic sheet material.

In some embodiments it may be necessary to subject the thin thermoplastic sheet material 14 to multiple passes through the apparatus 10 in order to microaperture the thin sheet material 14. In such situations the thin sheet material 14 will initially only be thinned in the pattern of the pattern anvil's raised areas. However, after two or more passes through the apparatus 10, with the thin thermoplastic sheet material 14 being aligned in the same configuration with respect to the pattern anvil, microapertures may be formed. Essentially what is happening in these situations is that the thin thermoplastic sheet material 14 is repeatedly thinned by repeated application of ultrasonic vibrational force until such time as microapertures are formed. Alternatively, the fine mesh wire diameter size may be increased with the consequent decrease in mesh count. Increasing the wire diameter size of the fine mesh screen 32 increases the liklihood that microapertures will be formed.

Another feature of the present invention is the fact that the microapertures can be formed in a predesignated area or areas of the thin thermoplastic sheet material 14. This can be accomplished in a number of ways. For example, the thin thermoplastic sheet material 14 may be subjected to ultrasonic vibrations only at certain areas of the sheet material, thus, microaperturing would occur only in those areas. Alternatively, the entire thin thermoplastic sheet material could be subjected to ultrasonic vibrations with the pattern anvil having raised areas only at certain locations and otherwise being flat. Accordingly, the thin thermoplastic sheet material would be microapertured only in those areas which correspond to areas on the pattern anvil having raised areas.

It should also be noted that some limitation exists in the number of microapertures which can be formed in a given thin thermoplastic sheet material 14 on a single application of vibrational energy, i.e. a single pass through the apparatus if a wire mesh screen is used as the pattern anvil. This follows from the fact that, as was stated above, the height of the raised areas must exceed the thickness of the thin thermoplastic sheet material 14 in conjunction with the fact that, generally, as the mesh count increases the height of the raised areas or knuckles decreases. In such situations, if the number of microapertures desired per unit area is greater than the number which can be formed in one pass through the apparatus, multiple passes are necessary with the alignment of the thin thermoplastic sheet material 14 with respect to the raised areas being altered or shifted slightly on each pass.

Generally speaking the area of each of the microapertures is greater than about ten square micrometers. That is the area of each of the microapertures may range from at least about 10 square micrometers to about 100,000 square micrometers. For example, the area of each of the formed microapertures may generally range from at least about 10 square micrometers to about 10,000 square micrometers. More particularly, the area of each of the formed microapertures may generally range from at least about 10 square micrometers to about 1,000 square micrometers. Even more particularly, the area of each of the formed microapertures may generally range from at least about 10 square micrometers to about 100 square micrometers.

A number of important observations about the process may now be made. For example, it should be understood that the presence of the fluid 62 is highly important to the present inventive process which uses the fluid 62 as a coupling agent. Because a coupling agent is present, the microapertures are punched through the thin sheet material 14 as opposed to being formed by melting. Additionally, the presence of the shim plate 30 or its equivalent is necessary in order to provide an anvil mechanism against which the thin thermoplastic sheet material 14 may be worked, that is apertured, by the action of the tip 54 of the ultrasonic horn 50. Because the vibrating tip 54 of the ultrasonic horn 50 is acting in a hammer and anvil manner when operated in conjunction with the heavy duty mesh screen 28/shim plate 30/fine wire mesh 32 combination, it should be readily recognized that a certain degree of tension must be placed upon the transport mechanism 22 by the downward displacement of the ultrasonic horn 50. If there is little or no tension placed upon the transport mechanism 22, the shim plate 30 cannot perform its function as an anvil and microaperturing generally does not occur. Because both the shim plate 30 and the fine mesh pattern wire 32 form the resistance that the ultrasonic horn 50 works against, they may be collectively referred to as a pattern anvil combination. It should be easily recognized by those in the art that the function of the pattern anvil can be accomplished by other arrangements than the heavy duty mesh screen 28/shim plate 30/fine mesh screen 32 combination. For example, the pattern anvil could be a flat plate with raised portions acting to direct the microaperturing force of the ultrasonic horn 50. Alternatively, the pattern anvil could be a cylindrical roller having raised areas. If the pattern anvil is a cylindrical roller with raised areas, it is desirable for the pattern anvil to be wrapped or coated with or made from a resilient material. Where the pattern anvil is a mesh screen the resiliency is provided by the fact that the screen is unsupported directly below the point of application of ultrasonic vibrations to the mesh screen.

As a result of the microaperturing process, the edge length of the thin thermoplastic sheet material may be increased by at least about 100 percent as compared to the sheet's edge length prior to microaperturing. For example, the edge length of the thin thermoplastic sheet material may be increased by at least about 500 percent as compared to the sheet's edge length prior to microaperturing. More particularly, the edge length of the thin thermoplastic sheet material may be increased by at least about 1,500 percent as compared to the sheet's edge length prior to microaperturing. Even more particularly, the edge length of the thin thermoplastic sheet material may be increased by at least about 3,000 percent as compared to the sheet's edge length prior to microaperturing.

The invention will now be discussed with regard to specific examples which will aid those of skill in the art in a full and complete understanding thereof.

CONTROLS

Prior to utilizing the present process to microaperture exemplary thin thermoplastic sheet materials, the hydrohead and water vapor transmission rate (wvtr) of the selected materials were measured. The different thermoplastic sheet materials that were chosen were:

(1) a 0.5 mil thick polyethylene film obtained from the Edison Company of Plainfield, New Jersey under the trade designation "S/E 702";

(2) a 0.8 mil thick film manufactured by the Rexene Corporation of Dallas, Texas under the trade designation "Rextac" is believed to be a blend of 80%, by weight, of polypropylene manufactured by the Exxon Chemical Co. under the trade designation "Exxon 3445" and 20%, by weight, of an amorphous polyalphaolefin;

(3) a 0.48 mil thick polyester film obtained from the Pilcher Hamilton Corporation of Broadview, Illinois, under the trade designation "PHanex IHC".

(4) a 0.6 mil thick polypropylene film obtained from the Edison Company of Plainfield, New Jersey under the trade designation "KS0032P";

(5) a 1.4 mil thick filled film which is loaded with 50-60%, by weight, (40% by volume) calcium carbonate particles obtained from the DuPont Chemical Company of Canada under the trade designation "Evlon";

(6) a 1.0 mil thick polyethylene film filled with carbon particles. This material was cut from a trash bag purchased from the K Mart Corporation of Troy, Michigan; and

(7) a 1.0 mil thick polyether polyurethane film obtained from the Stevens Company of Northhampton, Mass. under the trade designation "XPR-824".

The hydrohead of each of these materials was in excess of 137 centimeters of water. (Two measurements were made for each material. This is the maximum hydrohead measurable by our equipment.)

The average of three wvtr measurements of the Edison polyethylene was 41.7 grams per square meter per day. The average of three wvtr measurements of the Rexene blend was 31.25 grams per square meter per day. The average of three wvtr measurements of the PHanex polyester was 20.8 grams per square meter per day. The average of two (one test failed) wvtr measurements of the Edison polypropylene was 15.6 grams per square meter per day. Because the Dupont Evlon was believed to be breathable, nine wvtr measurements of the DuPont Evlon were made for greater accuracy. The average of these nine measurements was 201.4 grams per square meter per day. The average of three wvtr measurements of the K Mart carbon filled polyethylene was 0 grams per square meter per day. The average of three wvtr measurements of the Stevens polyether polyurethane was 448 grams per square meter per day.

EXAMPLE I

A sheet of 0.5 mil thick Edison polyethylene film having the trade designation S/E 702 was cut into a length of about 11 inches and a width of about 8.5 inches. As was stated above, the hydrohead of the S/E 702 sheet prior to hydrosonic treatment was measured as being greater than 137 centimeters of water. The sample was subjected to hydrosonic treatment in accordance with the present invention.

A model 1120 power supply obtained from the Branson Company of Danbury, Connecticut, was utilized. This power supply, which has the capacity to deliver 1,300 watts of electrical energy, was used to convert 115 volt, 60 cycle electrical energy to 20 kilohertz alternating current. A Branson type J4 power level control, which has the ability to regulate the ultimate output of the model 1120 power supply from 0 to 100%, was connected to the model 1120 power supply. In this example, the power level control was set at 100%. The actual amount of power consumed was indicated by a Branson model A410A wattmeter. This amount was about 750 watts.

The output of the power supply was fed to a model 402 piezoelectric ultrasonic transducer obtained from the Branson Company. The transducer converts the electrical energy to mechanical movement. At 100% power the amount of mechanical movement of the transducer is about 0.8 micrometers.

The piezoelectric transducer was connected to a mechanical movement booster section obtained from the Branson Company. The booster is a solid titanium metal shaft with a length equal to one-half of the wave length of the 20 kilohertz resonant frequency. Boosters can be machined so that the amount of mechanical movement at their output end is increased or decreased as compared to the amount of movement of the transducer. In this example the booster increased the amount of movement and has a gain ratio of about 1:2.5. That is, the amount of mechanical movement at the output end of the booster is about 2.5 times the amount of movement of the transducer.

The output end of the booster was connected to an ultrasonic horn obtained from the Branson Company. The horn in this example is made of titanium with a working face of about 9 inches by about 1/2 inch. The leading and trailing edges of the working face of the horn are each curved on a radius of about 1/8 inch. The horn step area is exponential in shape and yields about a two-fold increase in the mechanical movement of the booster. That is, the horn step area has about a 1:2 gain ratio. The combined increase, by the booster and the horn step area, in the original mechanical movement created by the transducer yields a mechanical movement of about 4.0 micrometers.

The forming table arrangement included a small forming table which was utilized to transport and support the Edison polyethylene film to be microapertured. The forming table included two 2-inch diameter idler rollers which were spaced about 12 inches apart on the surface of the forming table. A transport mesh belt encircles the two idler rollers so that a continuous conveying or transport surface is created. The transport mesh belt is a square weave 20 x 20 mesh web of 0.020 inch diameter plastic filaments. The belt is about 10 inches wide and is raised above the surface of the forming table.

The transducer/booster/horn assembly, hereinafter the assembly, is secured in a Branson series 400 actuator. When power is switched on to the transducer, the actuator, by means of a pneumatic cylinder with a piston area of about 4.4 square inches, lowers the assembly so that the output end of the horn contacts the Edison polyethylene film which is to be microapertured. The actuator also raises the assembly so that the output end of the horn is removed from contact with the Edison polyethylene film when power is switched off.

The assembly is positioned so that the output end of the horn is adapted so that it may be lowered to contact the transport mesh belt between the two idler rollers. An 8-inch wide 0.005-inch thick stainless steel shim stock having a length of about 60 inches was placed on the plastic mesh transport belt to provide a firm support for a pattern screen which is placed on top of the stainless steel shim. In this example the pattern screen is a 250 by 250 mesh wire size weave stainless steel screen. The Edison polyethylene film which was to be microapertured was then fastened onto the pattern wire using masking tape.

The forming table arrangement also included a fluid circulating system. The circulating system includes a fluid reservoir tank, a fluid circulating pump which may conveniently be located within the tank, associated tubing for transporting the fluid from the tank to a slotted boom which is designed to direct a curtain of fluid into the juncture of the output end of the horn and the Edison polyethylene film which is to be microapertured.

In operation, the assembly was positioned so that the output end of the horn was at an angle of from about 10 to 15 degrees to the edison polyethylene film to be microapertured. Accordingly, a wedge shaped chamber was formed between the output end of the horn and the Edison polyethylene film to be microapertured. It is into this wedge shaped chamber that the fluid, in this example water, at room temperature, was directed by the slotted boom.

It should be noted that the actuator was positioned at a height to insure that, when the assembly is lowered, the downward movement of the output end of the horn is stopped by the tension of the transport mesh before the actuator reaches the limit of its stroke. In this example, actuating pressure was adjusted to 8 pounds per square inch as read on a pressure gauge which is attached to the pneumatic cylinder of the actuator. This adjustment results in a total downward force of 35.2 pounds. 8 psi times 4.4 square inches of piston area equals 35.2 pounds of force.)

The sequence of operation was (1) the fluid pump was switched on and the area where the output end of the horn was to contact the Edison polyethylene film was flooded with water; (2) the transport mesh conveyor system was switched on and the polyethylene film started moving at 14.5 feet per minute; and (3) power to the assembly was supplied and the assembly was lowered so that the output end of the horn contacted the polyethylene film while the sample continued to pass under the output end of the horn until the end of the sample was reached. The reading on the A410A wattmeter during the process is an indication of the energy required to maintain maximum mechanical movement at the output end of the horn while working against the combined mass of the water, the polyethylene film, the pattern wire, the shim stock, and the transport wire.

This example yielded a microapertured polyethylene film having a maximum microaperture density of about 31,000 microapertures per square inch with the microapertures having an area of about 16 square micrometers. The average of three hydrohead values of the microapertured polyethylene film was measured as being about 21 centimeters of water and the average of three wvtr values of the microapertured polyethylene film was measured as being about 1,385 grams per square meter per day.

Figure IV is a photomicrograph of the thin polyethylene material microapertured in accordance with Example I.

ILLUSTRATIVE EXAMPLE

In order to illustrate the importance of the presence of the fluid as a coupling agent an illustrative example was run. This example attempted to duplicate Example I with the exception that no fluid was provided to the area where ultrasonic vibrations were being applied to the Edison polyethylene film. All process parameters of this illustrative example were the same as those of Example I with the exception that the line speed was seven (7) feet per minute and the actual amount of power consumed was indicated by the Branson model A410A wattmeter as about 800 watts.

Importantly, not only were no microapertures formed in the Edison polyethylene film and the film melted.

EXAMPLE II

The process of Example I was repeated with the exception that the line speed was 22 feet per minute and the watts consumed was about 800.

This example yielded a microapertured polyethylene film having a maximum microaperture density of about 31,000 microapertures per square inch with the microapertures having an area of about 15 square micrometers. The average of three hydrohead values of the microapertured polyethylene film was about 28 centimeters of water and the average of three wvtr values of the microapertured polyethylene film was measured as being about 1,083 grams per square meter per day.

EXAMPLE III

The process of Example I was repeated with the exception that the line speed was about 22.6 feet per minute, the fine mesh forming screen was 200 by 200 wires per inch and the watts consumed were about 800.

This example yielded a microapertured polyethylene film having a maximum microaperture density of about 20,000 microapertures per square inch with the microapertures having an area of about 12.3 square micrometers. The average of three hydrohead values of the microapertured polyethylene film was about 26 centimeters of water and the average of three wvtr values of the microapertured polyethylene film was about 1,052 grams per square meter per day.

EXAMPLE IV

The process of Example I was repeated with the exception that the line speed was 8.5 feet per minute, the fine mesh forming screen was a 325 by 325 wires per inch screen, the actuating pressure was increased to about 9 pounds per square inch and about 900 watts of energy was consumed.

This example yielded a microapertured polyethylene film having a maximum microaperture density of about 105,000 microapertures per square inch with the microapertures having an area of about 10 square micrometers. The average of two hydrohead values of the microapertured polyethylene film was measured as being about 49 centimeters of water and the average of three wvtr values of the microapertured polyethylene film was measured as being about 1,021 grams per square meter per day.

EXAMPLE V

The process of Example I was repeated with the exception that the line speed was 7 feet per minute, a 50%/50%, by volume, mixture of water and 2-propanol was used as the fluid, and about 800 watts of energy was consumed.

This example yielded a polyethylene film having large apertures which resulted in a hydrohead value (single reading) of about 17 centimeters of water. Because of the low hydrohead value, no wvtr value was obtained.

EXAMPLE VI

The process of Example V was repeated with the exception that the line speed was 24.5 feet per minute, the actuating pressure was decreased to about 3 pounds per square inch and 600 watts of energy was consumed.

This example failed to microaperture the film. However, one hydrohead value was measured and was about 78 centimeters of water and the average of three wvtr values of the film was about 52 grams per square meter per day. The increase in wvtr and decrease in hydrohead leads to the belief that the film, while not microapertured, was microarea thinned.

EXAMPLE VII

The process of Example I was repeated with the exception that the line speed was about 25 feet per minute, the actuating force was about 4 pounds per square inch, ethylene glycol was used as the fluid, and about 800 watts of energy was consumed.

This example yielded a microapertured polyethylene film having a maximum microaperture density of about 31,000 microapertures per square inch with the microapertures having an area of about 40 square micrometers. One hydrohead value was measured as being about 49 centimeters of water and the average of three wvtr values of this film was about 188 grams per square meter per day.

EXAMPLE VIII

The process of Example VII was repeated with the exception that the line speed was about 24.8 feet per minute, the actuating force was about 3 pounds per square inch, a 200 by 200 stainless steel fine mesh wire screen was used and about 750 watts of energy was consumed.

This example yielded a microapertured polyethylene film having a maximum microaperture density of about 20,000 microapertures per square inch with the microapertures having an area of about 431 square micrometers. One hydrohead value was measured as being about 27 centimeters of water and the one wvtr value obtained for this film was about 1,313 grams per square meter per day.

EXAMPLE IX

The process of Example I was repeated with the exception that the Rexene Rextec blend film was substituted for the Edison polyethylene, the line speed was about 7.6 feet per minute and the watts consumed were about 950.

This example yielded a microapertured film blend having a maximum microaperture density of about 31,000 microapertures per square inch with the microapertures having an area of in the range of about 16-150 square micrometers. The average of two hydrohead values of the microapertured blended film was measured as being about 22 centimeters of water and the average of three wvtr values of the microapertured blended film was measured as being about 1,375 grams per square meter per day.

EXAMPLE X

The process of Example VII was repeated with the exception that the line speed was about 11.5 feet per minute.

This example yielded a microapertured film blend having a maximum microaperture density of about 31,000 microapertures per square inch with the microapertures having an area in the range of about 12-80 square micrometers. The average of two hydrohead values of the microapertured blended film was measured as being about 30 centimeters of water and the average of three wvtr values of the microapertured blended film was measured as being about 1,490 grams per square meter per day.

EXAMPLE XI

The process of Example I was repeated with the exception that the carbon filled K Mart polyethylene film was substituted for the Edison polyethylene, the line speed was about 8.2 feet per minute and the watts consumed were about 800-875.

This example failed to microaperture the film. The hydrohead and wvtr remained substantially unaffected as compared to the control.

EXAMPLE XII

The process of Example XI was repeated with the exception that a 200 by 200 stainless steel fine wire mesh screen was used and the watts consumed were about 800-850.

This example yielded a microapertured carbon filled polyethylene film having a maximum microaperture density of about 20,000 microapertures per square inch with the microapertures having an area of about 45 square micrometers. The average of three hydrohead values of the microapertured carbon filled film was about 33 centimeters of water and the average of three wvtr values of the microapertured carbon filled film was about 104 grams per square meter per day.

EXAMPLE XIII

The process of Example XII was repeated with the exception that the line speed was reduced to about 4.2 feet per minute, a 250 by 250 stainles steel fine wire mesh screen was used and the actuating pressure was increased to about 10 pounds per square inch. Additionally, the watts consumed were about 900-1,000.

While no microapertures were noticed upon examination of the film, the average of three hydrohead values of the carbon filled film was about 20 centimeters of water and the average of three wvtr values of the carbon filled film was about 990 grams per square meter per day. The increase in wvtr and decrease in hydrohead leads to the belief that, while microapertures were not located in the areas observed, they were present elsewher in the sample.

EXAMPLE XIV

The process of Example XII was repeated.

This example yielded a microapertured carbon filled polyethylene film having a maximum microaperture density of about 20,000 microapertures per square inch with the microapertures having an area of about 45 square micrometers. The average of two hydrohead values of the microapertured carbon filled film was about 33 centimeters of water and the average of three wvtr values of the microapertured carbon filled film was about 1,063 grams per square meter per day. No explanation is presently know for the variation in wvtr results with those of Example X, perhaps variations in the film were responsible.

EXAMPLE XV

The process of Example I was repeated with the exception that the Dupont Evlon was substituted for the Edison polyethylene. Additionally, the line speed was about 7 feet per minute and the watts consumed were about 900.

This example yielded a microapertured film having a maximum microaperture density of about 31,000 microapertures per square inch with the microapertures having an area of about 300 square micrometers. The value of one hydrohead measurement taken was about 29 centimeters of water and the average value of two wvtr measurements made of the microapertured film was about 1,531 grams per square meter per day.

EXAMPLE XVI

The process of Example I was repeated with the exception that the PHanex polyester was substituted for the Edison polyethylene. Additionally, the line speed was about 5.3 feet per minute and the watts consumed were about 800.

This example failed to microaperture the film. The three hydrohead values obtained were still in excess of 137 centimeters of water. The average of two wvtr values for this film was about 78 grams per square meter per day. The increase in wvtr leads to the belief that the film, while not microapertured, was microarea thinned.

EXAMPLE XVII

The process of Example XVI was repeated with the exception that the line speed was about 4.4 feet per minute, 1,1,1 trichloroethane was used as the fluid, the actuating pressure was about 13 pounds per square inch, a 200 by 200 stainless steel fine mesh wire screen was used and the watts consumed were about 1,000.

This example yielded a microapertured film having a maximum microaperture density of about 20,000 microapertures per square inch with the microapertures having an area of about 86 square micrometers. The value of one hydrohead measurement taken was about 32 centimeters of water and the value of one wvtr measurement made of the microapertured film was about 969 grams per square meter per day.

EXAMPLE XVIII

The process of Example XVI was repeated with the exception that the line speed was about 4.3 feet per minute, a 200 by 200 fine wire mesh stainless steel screen was used, carbon tetrachloride was used as the fluid and the watts consumed were about 1,150.

This example yielded a microapertured film having a maximum microaperture density of about 20,000 microapertures per square inch with the microapertures having an area of about 235 square micrometers. The value of one hydrohead measurement taken was about 30 centimeters of water and the value of one wvtr measurement made of the microapertured film was about 1,031 grams per square meter per day.

EXAMPLE XIX

The process of Example XVI was repeated with the exception that the line speed was about 15 feet per minute, a 120 by 120 fine wire mesh stainless steel screen was used, the actuating pressure was about 7 pounds per square inch and the watts consumed were about 900.

This example yielded a microapertured film having a maximum microaperture density of about 7,000 microapertures per square inch with the microapertures having an area of about 1,675 square micrometers. The average value of two hydrohead measurements taken was about 31 centimeters of water and the average value of three wvtr measurements made of the microapertured film was about 594 grams per square meter per day.

EXAMPLE XX

The process of Example XVI was repeated with the exception that the line speed was about 19.8 feet per minute, a 120 by 120 fine wire mesh stainless steel screen was used, the actuating pressure was about 7 pounds per square inch, the fluid was a mixture of 50% water and 50% isopropanol, by volume, and the watts consumed were about 800.

This example yielded a microapertured film having a maximum microaperture density of about 7,000 microapertures per square inch with the microapertures having an area of about 1,500 square micrometers. The value of one hydrohead measurement taken was about 33 centimeters of water and the value of one wvtr measurement made of the microapertured film was about 504 grams per square meter per day.

EXAMPLE XXI

The process of Example XVI was repeated with the exception that the line speed was about 9.4 feet per minute, a 200 by 200 fine wire mesh stainless steel screen was used, the actuating pressure was about 13 pounds per square inch, 1,1,1 trichloroethane was used as the fluid and the watts consumed were about 1,100.

This example yielded a microapertured film having a maximum microaperture density of about 20,000 microapertures per square inch with the microapertures having an area of about 86 square micromecers. The value of one hydrohead measurement taken was about 32 centimeters of water and the value of one wvtr measurement made of the microapertured film was about 977 grams per square meter per day.

EXAMPLE XXII

The process of Example XVI was repeated with the exception that the line speed was about 4.3 feet per minute, a 200 by 200 fine wire mesh stainless steel screen was used, the actuating pressure was about 14 pounds per square inch, carbon tetrachloride was the fluid and the watts consumed were about 1,100.

This example yielded a microapertured film having a maximum microaperture density of about 20,000 microapertures per square inch with the microapertures having an area of about 1,000 square micrometers. The value of one hydrohead measurement taken was about 30 centimeters of water and the value of one wvtr measurement made of the microapertured film was about 1,040 grams per square meter per day.

EXAMPLE XXIII

The process of Example I was repeated with the exception that the Stevens polyether polyurethane film was substituted for the Edison polyethylene, the line speed was about 3.8 feet per minute, a 120 by 120 fine wire mesh stainless steel screen was used, the actuating pressure was about 10 pounds per square inch and the watts consumed were about 900.

This example yielded a microapertured film having a maximum microaperture density of about 7,000 microslits per square inch. The value of one hydrohead measurement taken was about 75 centimeters of water and the average value of two wvtr measurements made of the microapertured film was about 1,656 grams per square meter per day.

EXAMPLE XXIV

The process of Example I was repeated with the exception that the Edison polypropylene film was substituted for the Edison polyethylene, the line speed was about 2.7 feet per minute, the actuating pressure was about 10 pounds per square inch and the watts consumed were about 900-950.

This example yielded a microapertured film having a maximum microaperture density of about 31,000 microapertures per square inch with the microapertures having an area of about 122 square micrometers. The value of one hydrohead measurement taken was about 20 centimeters of water and the average value of two wvtr measurements made of the microapertured film was about 1,016 grams per square meter per day.

A series of examples was conducted to examine the effects of the various parameters on edge length change. In all of these examples the sheet material was the Edison polyethylene obtained under the trade designation "S/E 702". In all of these examples water was used as the fluid. The parameters and the percent edge length change are reported below in Table I for ease of comparison.

TABLE I

| Example | Line Speed[1] | Actuating Pressure[2] | Mesh[3] | Watts Consumed | No. of Apertures[4] | Size of Apertures[5] | Hydro-Head[6] | WVTR[7] | % Edge Increase |
|---|---|---|---|---|---|---|---|---|---|
| XXV | 5.3 | 16 | 120 | 1,200 | 7,000 | 415 | 16 | 1,078 | 511 |
| XXVI | 5.3 | 16 | 200 | 1,200 | 20,000 | 157 | 16 | 1,078 | 872 |
| XXVII | 5.3 | 16 | 250 | 1,200 | 31,000 | 379 | 19 | 1,406 | 2,113 |
| XXVIII | 5.3 | 16 | 325 | 1,200 | 50,000 | 227 | 16 | 1,180 | 3,089 |
| XXIX | 5.3 | 14 | 250 | 1,200 | 31,000 | 351 | 15 | 1,046 | 2,026 |
| XXX | 5.3 | 12 | 250 | 1,200 | 31,000 | 324 | 17 | 1,312 | 1,957 |
| XXXI | 5.3 | 10 | 250 | 1,200 | 31,000 | 209 | 15 | 1,063 | 1,750 |

[1] In feet per minute.

[2] In pounds per square inch.

[3] In all cases the mesh was a square mesh. Therefore, the number 120 would represent a mesh of 120 MD by 120 CD.

[4] Number per square inch.

[5] In square micrometers.

[6] Average of * readings.

[7] Average of two measurements.

While several examples which failed to produce microapertures have been included herein, others were encountered. It is believed that those of ordinary skill in the art will be able, with limited experimentation, if necessary, to determine the parameters necessary to produce microapertures in various materials.

The uses to which the microapertured thermoplastic sheet material of the present invention may be put are numerous. These include breathable outer cover stock for disposable diapers, fluid transfer coverstock

EP 0 535 579 A1

for feminine care products, filtration devices and breathable food wrap.

It is to be understood that variations and modifications of the present invention may be made without departing from the scope of the invention. For example, in some embodiments the use of multiple ultrasonic horns aligned abreast or sequentially may be desirable. It is also to be understood that the scope of the present invention is not to be interpreted as limited to the specific embodiments disclosed herein, but only in accordance with the appended claims when read in light of the foregoing disclosure.

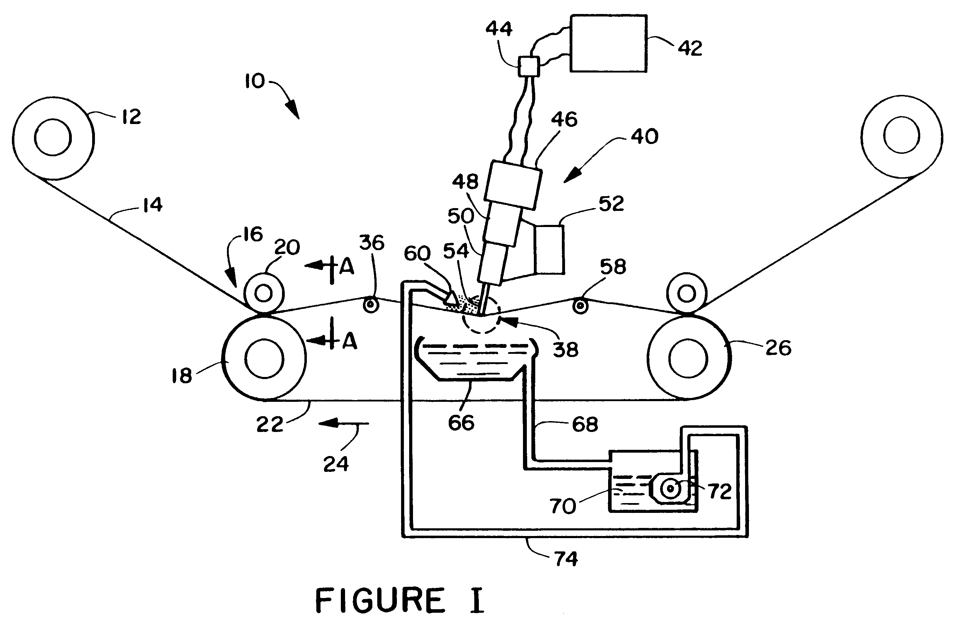

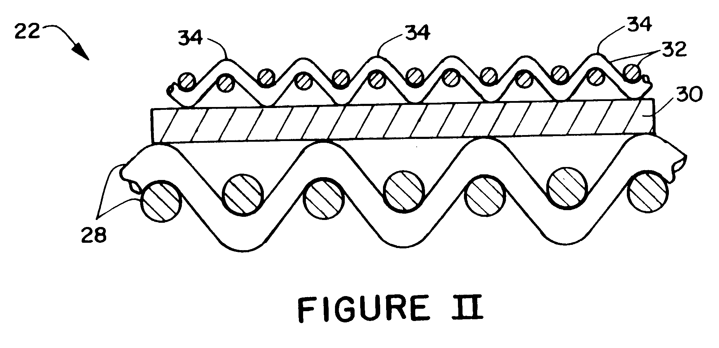

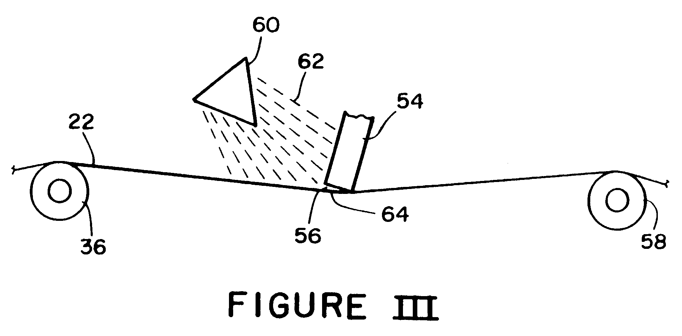

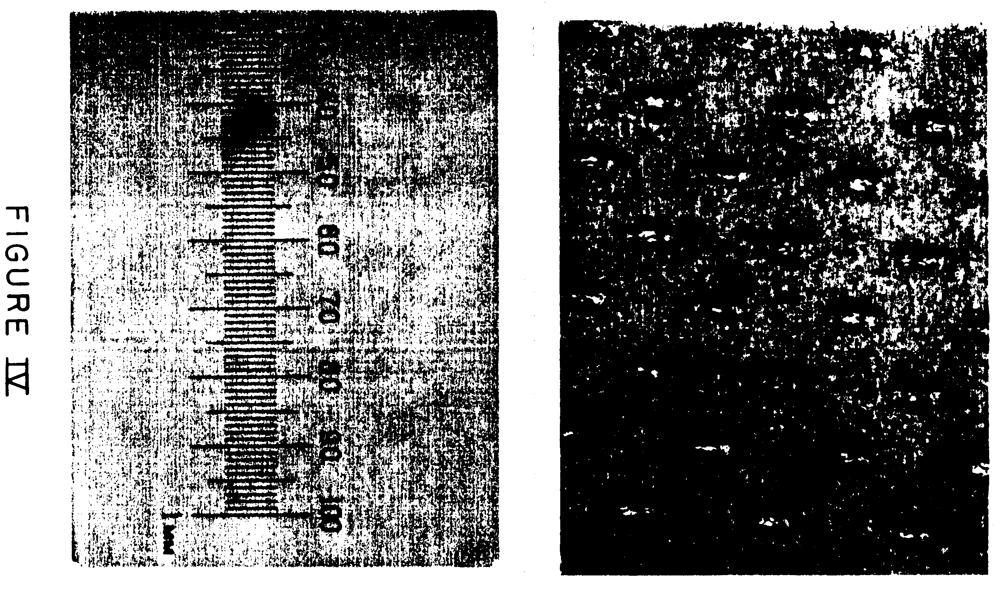

**Claims**

1. A microapertured thin thermoplastic sheet material having at least about 1,000 microapertures per square inch.

2. The microapertured thin thermoplastic sheet material of claim 1, having at least about 5,000 microapertures per square inch.

3. The microapertured thin thermoplastic sheet material of claim 1, having at least about 20,000 microapertures per square inch.

4. The microapertured thin thermoplastic sheet material of claim 1, having at least about 90,000 microapertures per square inch.

5. The microapertured thin thermoplastic sheet material of claim 1, having at least about 160,000 microapertures per square inch.

6. The microapertured thin thermoplastic sheet material according to claim 1, wherein the edge length of the sheet material is at least 100 percent greater than the edge length of the thin thermoplastic sheet material prior to microaperturing.

7. The microapertured thin thermoplastic sheet material according to claim 1, wherein the edge length of the sheet material is at least 1,500 percent greater than the edge length of the thin thermoplastic sheet material prior to microaperturing.

8. The microapertured thin thermoplastic sheet material according to claim 1, wherein the edge length of the sheet material is at least 3,000 percent greater than the edge length of the thin thermoplastic sheet material prior to microaperturing.

9. The microapertured thin thermoplastic sheet material of claim 1, wherein the average thickness of the thermoplastic sheet material is at least about 0.25 mil.

10. The microapertured thin thermoplastic sheet material of claim 1, wherein the average thickness of the thermoplastic sheet material is from about 0.25 mil to about 5 mils.

11. The microapertured thin thermoplastic sheet material of claim 1, wherein the average thickness of the thermoplastic sheet material is from about 0.25 mil to about 2 mils.

12. The microapertured thin thermoplastic sheet material of claim 1, wherein the average thickness of the thermoplastic sheet material is from about 0.5 mil to about 1 mil.

13. The microapertured thin thermoplastic sheet material of claim 1, wherein the area of each of the formed microapertures generally ranges from at least about 10 square micrometers to about 100,000 square micrometers.

14. The microapertured thin thermoplastic sheet material of claim 1, wherein the area of each of the formed microapertures generally ranges from at least about 10 square micrometers to about 10,000 square micrometers.

15. The microapertured thin thermoplastic sheet material of claim 1, wherein the area of each of the formed microapertures generally ranges from at least about 10 square micrometers to about 5,000 square

17

micrometers.

16. The microapertured thin thermoplastic sheet material of claim 1, wherein the area of each of the formed microapertures generally ranges from at least about 10 square micrometers to about 1,000 square micrometers.

17. The microapertured thin thermoplastic sheet material of claim 1, wherein the microaperturing is confined to a predesignated area or areas of the thin thermoplastic sheet material.

18. The microapertured thin thermoplastic sheet material of claim 1, wherein the thermoplastic material is a water insoluble material and the hydrohead of the sheet material is at least about 15 centimeters of water.

19. The microapertured thin thermoplastic sheet material of claim 1, wherein the thermoplastic material is a water insoluble material and the hydrohead of the sheet material is at least about 35 centimeters of water.

20. The microapertured thin thermoplastic sheet material of claim 1, wherein the thermoplastic material is a water insoluble material and the hydrohead of the sheet material is at least about 45 centimeters of water.

21. The microapertured thin thermoplastic sheet material of claim 1, wherein the thermoplastic material is a water insoluble material and the hydrohead of the sheet material is at least about 55 centimeters of water.

22. The microapertured thin thermoplastic sheet material of claim 1, wherein the thermoplastic material is a water insoluble material and the hydrohead of the sheet material is at least about 75 centimeters of water.

23. The microapertured thin thermoplastic sheet material of claim 1, wherein the thermoplastic material is a water insoluble material and the water vapor transmission rate of the sheet material is at least about 200 grams per square meter per day.

24. The microapertured thin thermoplastic sheet material of claim 1, wherein the thermoplastic material is a water insoluble material and the water vapor transmission rate of the sheet material is at least about 500 grams per square meter per day.

25. The microapertured thin thermoplastic sheet material of claim 1, wherein the thermoplastic material is a water insoluble material and the water vapor transmission rate of the sheet material is at least about 1,000 grams per square meter per day.

26. The microapertured thin thermoplastic sheet material of claim 1, wherein the thermoplastic material is selected from one or more of the group consisting of one or more of polyolefins, polyesters, nylons, caprolactams, polyvinyl alcohol or thermoplastic elastomers.

27. Them microapertured thin thermoplastic sheet material of claim 24, wherein the polyolefin is polyethylene.

28. A microapertured, substantially water insoluble, thin, thermoplastic sheet material having a thickness of about 1 mil or less, said sheet material having:
    an edge length which is at least 500 percent greater than the edge length of the thin thermoplastic sheet material prior to microaperturing;
    a microaperture density of at least about 100,000 microapertures per square inch;
    a hydrohead of at least about 75 centimeters of water; a water vapor transmission rate of at least about 200; and
    wherein the area of each of said microapertures ranges generally from greater than about 10 square micrometers to less than about 1,000 square micrometers.

18

29. A microapertured, substantially water insoluble, thin, thermoplastic sheet material having a thickness of about 1 mil or less, said sheet material having:

an edge length which is at least 500 percent greater than the edge length of the thin thermoplastic sheet material prior to microaperturing;

a microaperture density of at least about 100,000 microapertures per square inch;

a hydrohead of at least about 75 centimeters of water; a water vapor transmission rate of at least about 200 ; and

wherein the area of each of said microapertures ranges generally from greater than about 10 square micrometers to less than about 100 square micrometers.

FIGURE I

EP 0 535 579 A1

FIGURE II

FIGURE III

EP 0 535 579 A1

FIGURE IV

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,X | US-A-3 966 519 (K.J. MITCHELL ET AL.)<br>* column 3, line 37 - column 4, line 43; figures 1-5 * | 1-29 | B26F1/26 |
| A | US-A-4 980 215 (K.M. SCHONBRUN)<br>* column 5, line 22 - line 27 * | 13-16 | |
| A | GB-A-1 253 664 (SCOTT PAPER CO.)<br>* page 1, left column, line 12 - line 26 *<br>* page 1, left column, line 46 - right column, line 62 *<br>* page 3, left column, line 23 - line 48 * | 1-5 | |
| A | US-A-4 438 167 (E.C.A. SCHWARZ)<br>* column 2, line 26 - line 59 *<br>* example 7 * | 1-8 | |
| A | US-A-4 778 644 (THE PROCTER & GAMBLE CO.)<br>* column 9, line 12 - line 16 *<br>* column 16, line 4 - line 12; figure 5 * | 1-5,9-12 | |
| A | EP-A-0 259 003 (EL PASO)<br><br>* page 3, line 13 - line 15 *<br>* examples 1,2; table * | 9-12, 23-25 | TECHNICAL FIELDS SEARCHED (Int. Cl.5)<br><br>B26F<br>B29C<br>D21F<br>D04H |

The present search report has been drawn up for all claims

| Place of search | Date of completion of the search | Examiner |
|---|---|---|
| THE HAGUE | 11 NOVEMBER 1992 | TOPALIDIS A. |